# EUROPEAN PATENT SPECIFICATION

(11) **EP 1 745 750 B1**
(45) Date of publication and mention of the grant of the patent: **21.01.2009**
(21) Application number: 06253840.0
(22) Date of filing: 21.07.2006
(51) Int. Cl.: A61B 17/11, A61B 17/115, A61F 2/00

(54) **Anastomotic ring applier for use in colorectal applications**
Gerät zum Einsetzen von Anastomoseringen zur Verwendung in kolorektalen Anwendungen
Applicateur de baques d'anastomose à utiliser dans des applications colorectales

(30) Priority: 22.07.2005 US 187658
(43) Date of publication of application: 24.01.2007
(73) Proprietor: ETHICON ENDO-SURGERY, INC., Cincinnati, Ohio 45242 (US)
(72) Inventor: Ortiz, Marc S., Milford, OH 45150 (US)
(74) Representative: Tunstall, Christopher Stephen

(56) References cited:
- EP-A- 1 520 531

## Description

### FIELD OF THE INVENTION

The present invention relates, in general, to surgery and, more particularly, to a device for performing a surgical procedure on the digestive system.

### BACKGROUND OF THE INVENTION

The percentage of the world population suffering from morbid obesity is steadily increasing. Severely obese persons may be susceptible to increased risk of heart disease, stroke, diabetes, pulmonary disease, and accidents. Because of the effects of morbid obesity on the life of the patient, methods of treating morbid obesity have been the subject of intense research.

One known method for treating morbid obesity includes the use of anastomotic rings. Devices for applying anastomotic rings are known in the art. Devices of this nature are commonly adapted to insert a compressed anastomotic ring to an anastomotic opening formed between proximate gastrointestinal tissue walls. These applier devices may utilize a ring deployment mechanism comprising an expansion element that is actuated once the compressed ring is placed in the anastomotic opening, causing the anastomotic ring to expand from its compressed, cylindrically-shaped position to an actuated, hollow rivet-shaped position.

There may be circumstances in which it would be advantageous to have an anastomotic ring applier compatible for use in colorectal applications. However, the elongated shaft of a conventional anastomotic ring applier device may be unsuitable for colorectal applications. In particular, a conventional anastomotic ring applier device may lack a desirable curvature and/or may not provide ideal leverage for such applications. In addition, the actuating mechanism used by a conventional ring applier may be unsuitable for use in colorectal applications. A conventional ring applier may be unsuitable or otherwise less than ideal for colorectal applications for a variety of other reasons.

Consequently, it may be desirable to have an anastomotic ring applier device that is adapted for use with colorectal applications. Specifically, it may be desirable to have an anastomotic ring applier device that comprises an elongated shaft that is suited for colorectal applications. Further, it may be desirable to have an anastomotic ring applier device that includes an actuating mechanism suited for use in colorectal applications.

EP 1 520 531 A discloses a surgical instrument for implanting an anastomotic ring device of the type set forth in the preamble of the accompanying claim 1.

### BRIEF SUMMARY OF THE INVENTION

According to a first aspect of the invention, there is provided a surgical instrument for implanting an anastomotic ring device of the type set forth in the accompanying claim 1. The rigid, curved element of the elongate shaft may enable the instrument to be used in colorectal applications, or otherwise facilitate such use, to deploy an anastomotic ring.

Further, preferred aspects are set out in the accompanying dependent claims.

### BRIEF DESCRIPTION OF THE FIGURES

The accompanying drawings, which are incorporated in and constitute a part of this specification, illustrate versions of the invention, and, together with the general description of the invention given above, and the detailed description of the versions given below, serve to explain the principles of the present invention.

FIGURE 1 is a perspective view of an anastomotic ring applier device.

FIGURE 2 is a partial perspective view of the distal portion of an anastomotic ring applier device holding an anastomotic ring in an unactuated position.

FIGURE 3 is a partial perspective view of the distal portion of the device of FIGURE 2 holding an anastomotic ring in the actuated position.

FIGURE 4 is a frontal view of an actuated anastomotic ring.

FIGURE 5 is a perspective view of the device of FIGURE 1, shown with the ring deployment mechanism in the actuated position.

FIGURE 6 is an exploded view of a distal portion of the device of FIGURE 1, shown with the ring deployment mechanism fully actuated.

FIGURE 7 is an exploded cross-sectional view of a proximal portion of the device of FIGURE 1.

FIGURE 8 is a cross-sectional view of a distal portion of the device of FIGURE 1.

FIGURE 9 is a cross-sectional view of a proximal portion of the device of FIGURE 1.

FIGURE 10 is a cross-sectional view of a distal portion of the device of FIGURE 1, with the ring deployment mechanism fully actuated.

FIGURE 11 is a cross-sectional view of a proximal portion of the device of FIGURE 1, with the actuating members in the actuated position.

FIGURE 12 is a cross-sectional view of a proximal portion of the device of FIGURE 1, taken along plane 12 of FIGURE 9.

### DETAILED DESCRIPTION OF EMBODIMENTS OF THE INVENTION

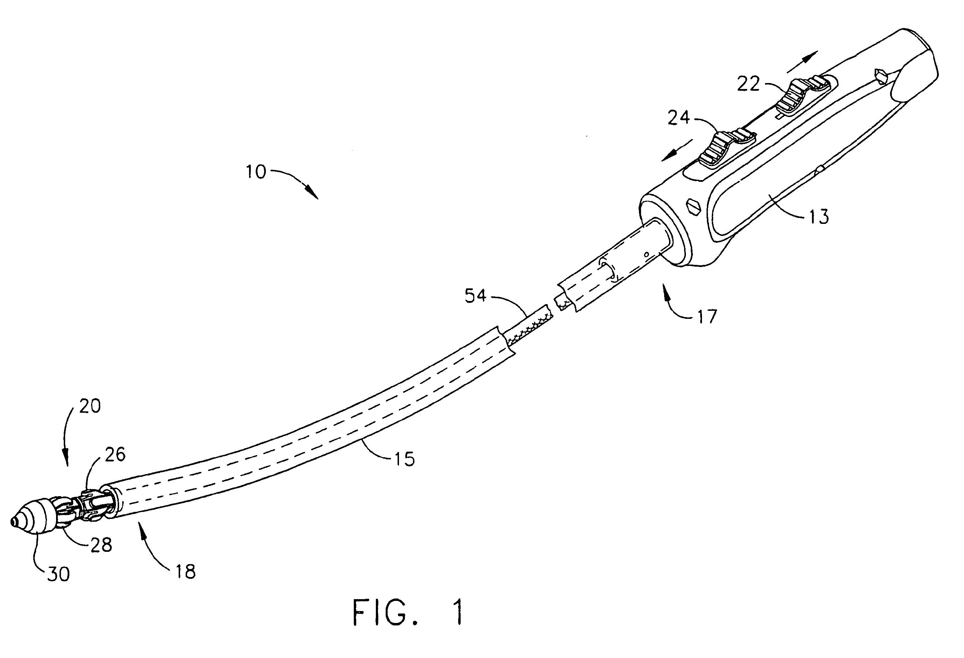

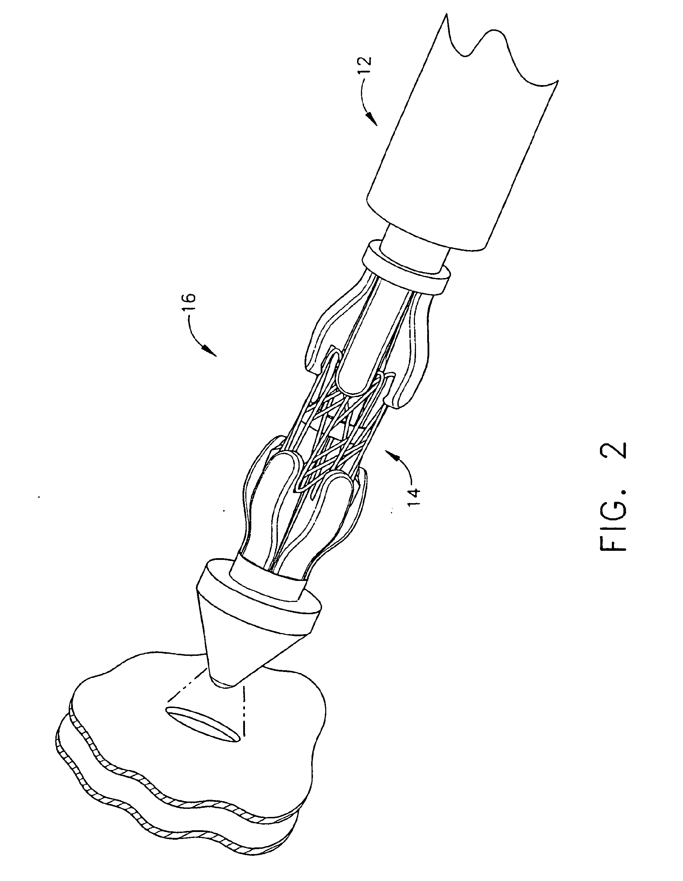

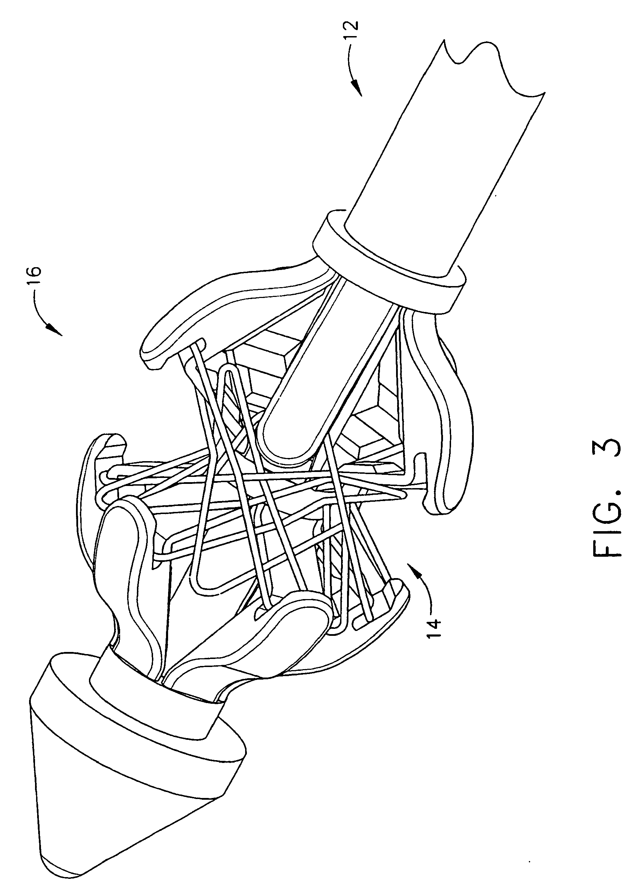

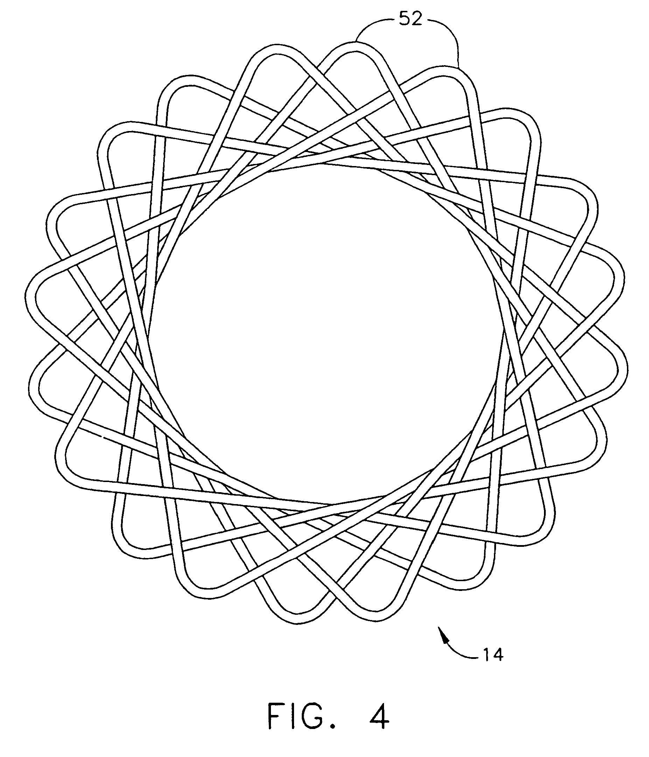

Turning to the Drawings, wherein like numerals denote like components throughout the several views, FIG. 1 depicts an applier 10 that is operable to deploy and actuate an anastomotic ring device (not pictured in FIG. 1) from a generally cylindrical shape to one having properties of a hollow rivet, or ring, capable of forming an anastomotic attachment at an anastomosis target site, such as in a bariatric gastric bypass of a morbidly obese patient. FIG. 2 depicts another applier 12. It will be appreciated that appliers 10. 12 may be used in a variety of ways, including but not limited to laparoscopically or endoscopically. Applier 12 is shown in FIG. 2 with an anastomotic ring 14 on a deployment mechanism 16. In FIG. 2, anastomotic ring 14 is shown in the compressed, cylindrically-shaped position. In FIG. 3, deployment mechanism 16 of applier 12 has moved anastomotic ring 14 to the actuated, hollow rivet-shaped position. FIG. 4 is a close-up view of anastomotic ring 14 in the actuated position. Anastomotic ring 14 may comprise a shape memory effect (SME) material, such as nitinol by way of example only, that further assists in actuation to an engaging hollow rivet shape. Other suitable anastomotic ring 14 materials will be apparent to those of ordinary skill in the art. An exemplary anastomotic ring 14 is described in detail in U.S. Patent Application Publ. No. US 2003/0032967 to Park et al.

It will be appreciated that the terms "proximal" and "distal" are used herein with reference to a clinician gripping a handle of applier 10. It will be further appreciated that for convenience and clarity, spatial terms such as "right", "left", "vertical" and "horizontal" are used herein with respect to the drawings. However, surgical instruments are used in many orientations and positions, and these terms are not intended to be limiting and absolute. In addition, aspects of the invention have application to surgical procedures performed endoscopically and laparoscopically, as well as an open procedure or other procedures. Use herein of one of these or similar terms should not be construed to limit the present invention for use in only one category of surgical procedure.

Referring to FIGS. 1, 5, and 6, applier 10 comprises a handle 13 and an elongated shaft 15 having a proximal end 17 and a distal end 18. Handle 13 is connected by shaft 15 to a ring deployment mechanism 20. Handle 13 comprises an actuating member 22 operable to communicate actuating forces to ring deployment mechanism 20. In the present example, applier 10 further includes a second actuating member 24, which is optional. Actuating members 22, 24 comprise sliders. Second slider 24 is located distal of first slider 22. Those of ordinary skill in the art will appreciate, however, that handle 13 may have any suitable number of actuating members 22, 24, that actuating members 22, 24 may take a variety of alternative forms, and that handle 13 and/or actuating members 22, 24 may be configured in a variety of alternative ways.

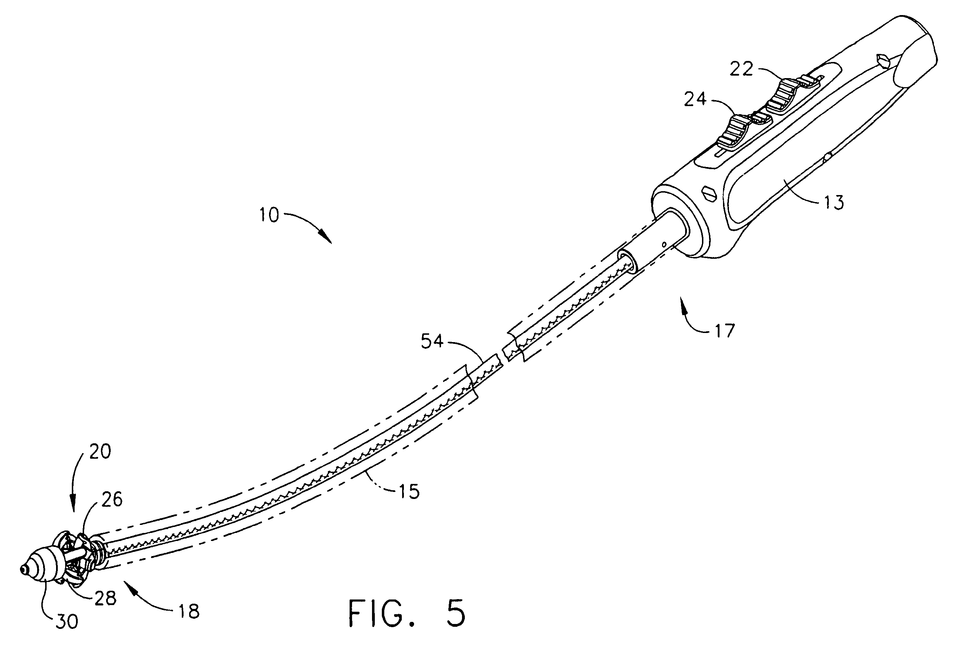

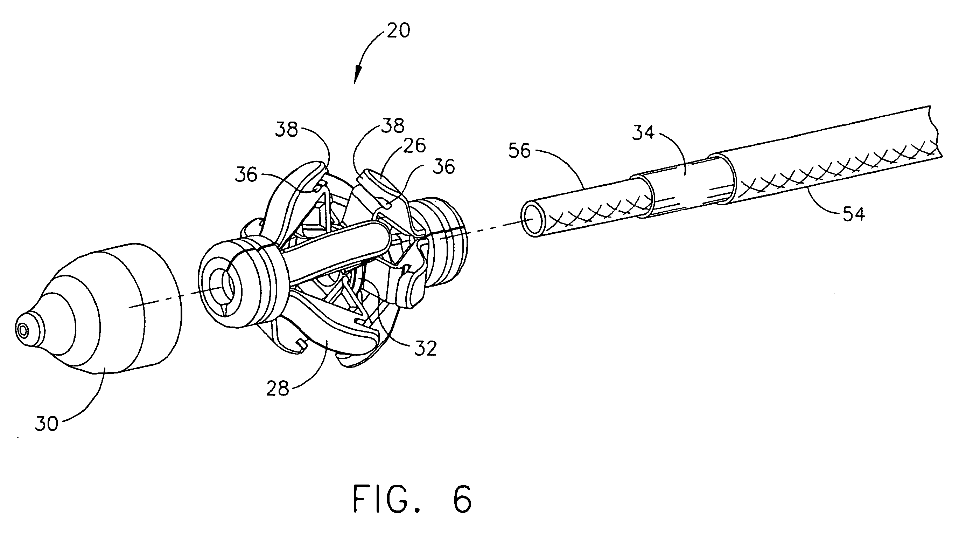

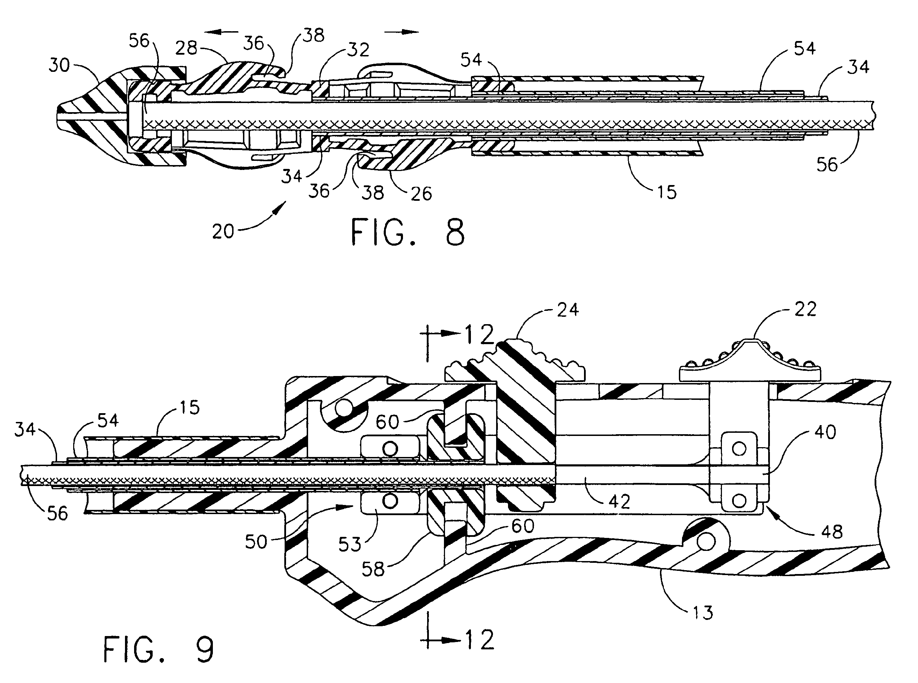

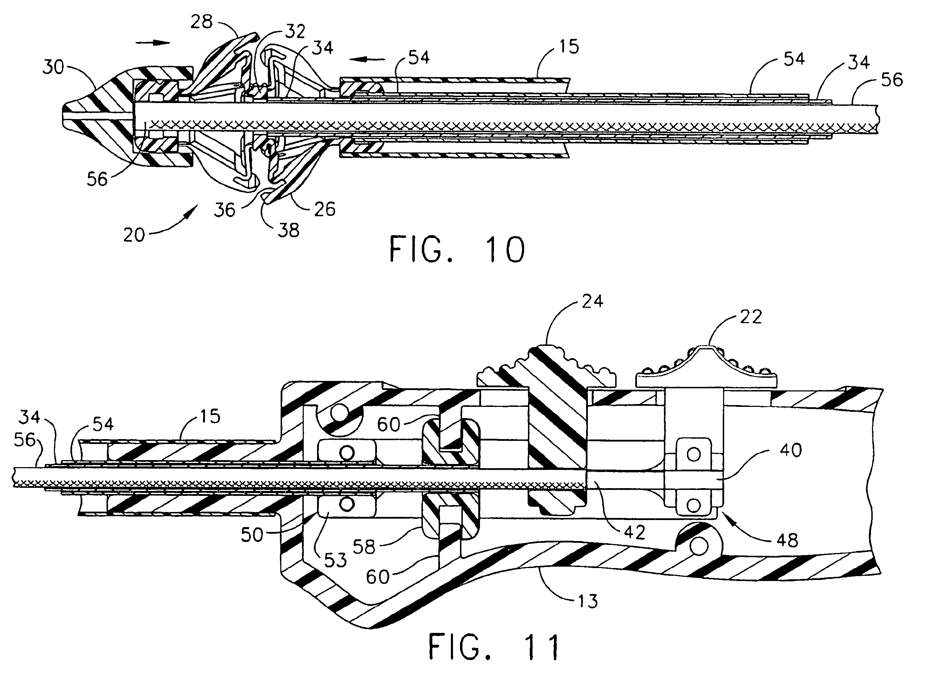

As shown in FIG. 5, sliders 22, 24 are adapted to slide from a first, unactuated position (FIG. 9) to an actuated position (FIG. 11) to actuate ring deployment mechanism 20. As best shown in FIG. 6, ring deployment mechanism 20 comprises proximal fingers 26 and distal fingers 28. Fingers 26, 28 are configured to hold an anastomotic ring by engaging petals 51 prior to and during deployment of the anastomotic ring, and release petals 51 upon deployment of the anastomotic ring. Applier 10 includes a tip 30 located distal of deployment mechanism 20. Tip 30 may attach to distal fingers 26, and/or may be connected by a rod (not pictured) to handle 13.

In the present example, ring deployment mechanism 20 comprises a stationary mid-ring 32 (FIG. 8). Mid-ring 32 is held stationary by a ground tube 34. Proximal and distal fingers 26, 28 are each in a double-hinged relationship with mid-ring 32. Fingers 26, 28 each comprise gripping slots 36 configured to hold the anastomotic ring prior to ring deployment, as well as during intermediate stages of ring deployment. Fingers 26, 28 further comprise inwardly-directed tips 38 configured to allow the anastomotic ring to slide out of engagement with fingers 26, 28 when the ring is fully deployed. Proximal and distal fingers 26, 28 are adapted to receive a proximal and distal portion, respectively, of an anastomotic ring. Proximal and distal fingers 26, 28 are further adapted to move from a first, unactuated position (FIG. 8) toward mid-ring 32 to a second, actuated position (FIG. 10) to deploy the anastomotic ring. As fingers 26, 28 move toward mid-ring 32, they are configured to articulate outwardly in the manner of an umbrella due to their hinged relationship with mid-ring 32, moving the anastomotic ring from the compressed position to the deployed, rivet-shaped position. Of course, ring deployment mechanism 20 may comprise a variety of alternative components and/or configurations. Such alternatives will be apparent to those of ordinary skill in the art.

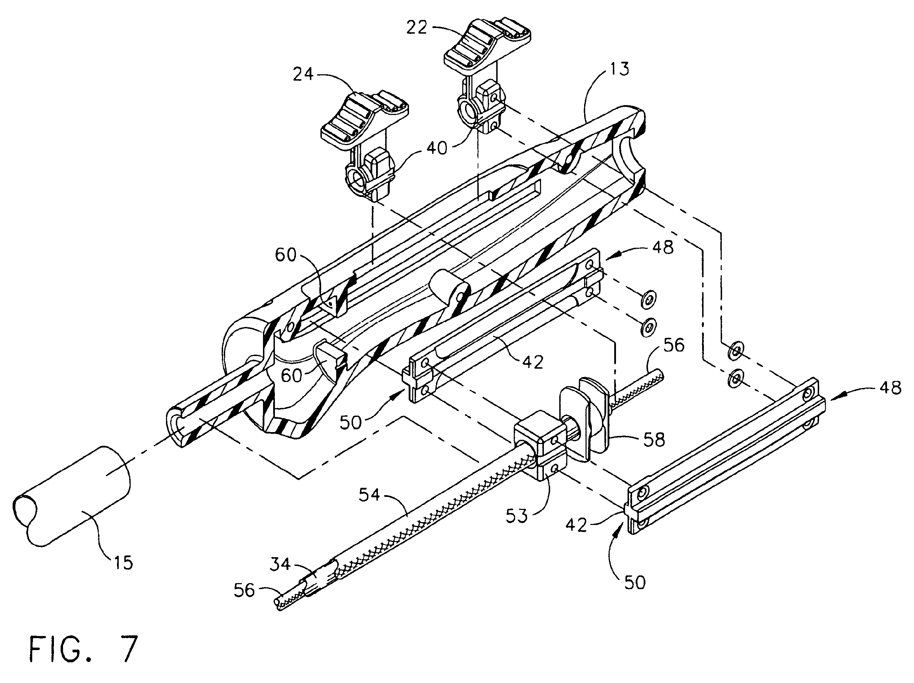

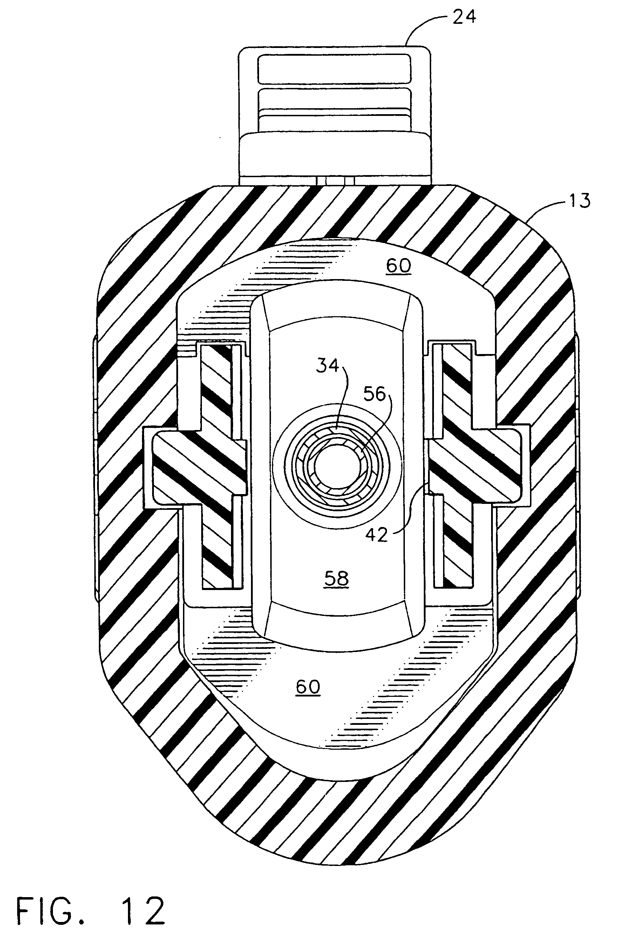

Referring now to FIGS. 7, 9, 11, and 12, a mechanism configured to transmit user input to ring deployment mechanism 20 is shown. In the present example, first deployment actuator 22 is operable to control proximal fingers 26, and second deployment actuator 24 is operable to control distal fingers 28. Alternatively, first deployment actuator 22 may control distal fingers 28 and second deployment actuator 24 may control proximal fingers 26. In the present example, first and second ring deployment actuators 22, 24 each comprise a pair of grooves 40 that are configured to slide on a track 42. Track 42 is further configured to slide within handle 13. Other suitable configurations will be apparent to those of ordinary skill in the art.

In the present example, first actuator 22 is fixedly attached to a proximal portion 48 of track 42. A distal portion 50 of track 42 is fixedly attached to a slider 53 that is slideably attached to handle 13. Slider 53 is connected to an outer tube 54. Longitudinal motion of first actuator 22 may thereby cause corresponding longitudinal motion of track 42, slider 53 and outer tube 54. Outer tube 54 is connected to proximal fingers 26. Outer tube 54 is thereby operable to communicate motion to proximal fingers 26.

Second actuator 24 is connected to an inner tube 56. Inner tube 56 extends longitudinally through ground tube 34, which extends longitudinally through outer tube 54. Inner tube 56 is connected to distal fingers 28. Inner tube 56 is thereby operable to communicate motion to distal fingers 28. In this manner, first actuator 22 is operable to control actuation of proximal fingers 26, and second actuator 24 is operable to control actuation of distal fingers 28. Ground tube 34, which is fixed to mid-ring 32 at the distal end of ground tube 34, is fixedly attached to anchor member 58 at the proximal end of ground tube 34. Anchor member 58 is configured to engage with bosses 60 in handle 13, thereby preventing relative motion between handle 13 and ground tube 34.

In the present example, it should be noted that although second actuator 24 is configured to slide on track 42, it is not statically attached to it. Therefore, longitudinal movement of track 42 due to motion of first actuator 22 will not cause longitudinal movement of second actuator 24. Those of ordinary skill in the art will appreciate, however, that a variety of alternative components and/or configurations may be used to effect actuation of distal fingers 28 and/or proximal fingers 26. By way of example only, one alternative configuration may include configuring first actuator 22 to be operable to control actuation of distal fingers 28, and configuring second actuator 24 to be operable to control actuation of proximal fingers 26. Other suitable variations will be apparent to those of ordinary skill in the art.

Ground tube 34 of the present example is rigid and comprises a curved portion. It will be appreciated that ground tube 34 is thus suitable for colorectal applications. Inner tube 56 and outer tube 54 are flexible, which allows them to follow the curve of ground tube 34 as they translate longitudinally in response to actuation of first and second actuators 22, 24. In one embodiment, inner and outer tubes 54, 56 are comprised of tightly woven wires. Other suitable features configurations of tubes 34, 54, and 56 will be apparent to those of ordinary skill in the art.

Having shown and described various embodiments and concepts of the invention, further adaptations of the methods and systems described herein can be accomplished by appropriate modifications by one of ordinary skill in the art without departing from the scope of the invention. Several of such potential alternatives, modifications, and variations have been mentioned, and others will be apparent to those skilled in the art in light of the foregoing teachings.

Accordingly, the invention is intended to embrace all such alternatives, modifications and variations as may fall within the scope of the appended claims and is understood not to be limited to the details of structure and operation shown and described in the specification and drawings. Additional advantages may readily appear to those skilled in the art.

## Claims

1. A surgical instrument (10; 12) for implanting an anastomotic ring device (14), comprising:
(i) a handle (13);
(ii) a ring deployment mechanism (16; 20) configured to receive an anastomotic ring (14), wherein the ring deployment mechanism is adapted to move the anastomotic ring (14)between an unactuated, generally cylindrical position and an actuated, hollow rivet-forming position;
(iii) an actuation mechanism (22, 54; 24, 56) operable to communicate actuating force to the ring-deployment mechanism (16; 20);
(iv) an elongate shaft (15) connecting the handle (13) to the ring deployment mechanism (16; 20) wherein the elongate shaft comprises one or more rigid, curved members (34); and
(v) an actuating member (22; 24) operable to actuate the ring deployment mechanism (16; 20)
**characterized in that** the actuating member (22; 24) is connected to the ring deployment mechanism (16; 20) by a flexible tube (54; 56).

2. The surgical instrument of Claim 1, wherein the flexible tube (54; 56) comprises woven wires.

3. The surgical instrument of Claim 1 or Claim 2, wherein the ring deployment mechanism (20) further comprises a stationary non-actuating element (32).

4. The surgical instrument of Claim 3, wherein the ring deployment mechanism (20) further comprises a translating element (26; 28).

5. The surgical instrument of Claim 4, wherein the translating element (26; 28) is in a hinged relationship with the stationary non-actuating element (32).

6. The surgical instrument of Claim 5, wherein the translating element (26; 28) is configured to move toward the stationary element (32) in response to actuating force, causing the translating element to articulate outwardly from the elongate shaft (15) to deploy a portion of the anastomotic ring (15).

7. The surgical instrument of any preceding claim, wherein the ring deployment mechanism (20) comprises a first plurality of fingers (26; 28) configured to receive the anastomotic ring (14), the fingers being moveable from a first position longitudinally aligned with the shaft (15) to a second position in which the fingers actuate outwardly from a longitudinal axis of the elongate shaft to actuate at least a portion of the anastomotic ring.

8. The surgical instrument of Claim 7, wherein the first plurality of fingers (26) are configured to receive and deploy a proximal portion of the anastomotic ring (14), wherein the ring deployment mechanism (20) further comprises a second plurality of fingers (28) configured to receive and deploy a distal portion of the anastomotic ring (14).

9. The surgical instrument of any of Claims 1 to 6, wherein the elongate shaft (15) comprises a proximal portion and a distal portion, wherein the proximal portion is attached to the handle (13), wherein the ring deployment mechanism (20) comprises a longitudinal end (28) and a center portion (32), wherein the ring deployment mechanism is positioned on the distal portion of the elongate shaft (15), wherein the ring deployment mechanism is configured to receive the anastomotic ring (14) in a compressed condition, and wherein the flexible connecting member (56) is operable to move the longitudinal end (28) of the ring deployment mechanism (20) toward the center portion (32) of the ring deployment mechanism to actuate at least a portion of the anastomotic ring (14).

## Patentansprüche

1. Chirurgisches Instrument (10;12) zum Implantieren einer Anastomoseringeinrichtung (14), umfassend:
(i) einen Handgriff (13);
(ii) einen Ringaussetzmechanismus (16; 20), der zur Aufnahme eines Anastomoseringes (14) gestaltet ist, wobei der Ringeinsetzmechanismus zum Bewegen des Anastomoseringes (14) zwischen einer nicht betätigten, allgemein zylindrischen Position und einer betätigten, hohlnietenförmigen Position gestaltet ist;
(iii) einen Betätigungsmechanismus (22, 54; 24, 56), der betreibbar ist, um eine Stellkraft auf den Ringaussetzmechanismus (16; 20) zu übertragen;
(iv) einen länglichen Schaft (15), der den Handgriff (13) mit dem Ringaussetzmechanismus (16; 20) verbindet, wobei der längliche Schaft ein oder mehrere starre(s), gekrümmte(s) Element(e) (34) aufweist;
(v) ein Betätigungselement (22; 24), das betreibbar ist, um den Ringaussetzmechanismus (16; 20) zu betätigen, **dadurch gekennzeichnet, dass** das Betätigungselement (22; 24) mit dem Ringaussetzmechanismus (16; 20) durch einen flexiblen Schlauch (54; 56) verbunden ist.

2. Chirurgisches Instrument nach Anspruch 1, **dadurch gekennzeichnet, dass** der flexible Schlauch (54; 56) gewebte Drähte umfasst.

3. Chirurgisches Instrument nach Anspruch 1 oder Anspruch 2, **dadurch gekennzeichnet, dass** der Ringaussetzmechanismus (20) ferner ein stationäres Nichtbetätigungselement (32) aufweist.

4. Chirurgisches Instrument nach Anspruch 3, **dadurch gekennzeichnet, dass** der Ringaussetzmechanismus (20) ferner ein Übertragungselement (26; 28) aufweist.

5. Chirurgisches Instrument nach Anspruch 4, **dadurch gekennzeichnet, dass** das Übertragungselement (26; 28) in einer Gelenkbeziehung mit dem stationären Nichtbetätigungselement (32) steht.

6. Chirurgisches Instrument nach Anspruch 5, **dadurch gekennzeichnet, dass** das Übertragungselement (26; 28) zum Bewegen in Richtung auf das stationäre Element (32) als Antwort auf eine Stellkraft konfiguriert ist, wodurch das Übertragungselement vom länglichen Schaft (15) zum Aussetzen eines Teils des Anastomoseringes (15) nach Außen schwenkt.

7. Chirurgisches Instrument nach einem der vorangegangenen Ansprüche, **dadurch gekennzeichnet, dass** der Ringaussetzmechanismus (20) eine erste Vielzahl von Fingern (26; 28) aufweist, die zur Aufnahme des Anastomoseringes (14) konfiguriert sind, wobei die Finger aus einer ersten Position, die longitudinal mit dem Schaft (15) ausgerichtet sind, zu einer zweiten Position bewegbar sind, in der sich die Finger von einer longitudinalen Achse des länglichen Schaftes nach außen bewegen, um wenigstens einen Teil des Anastomoseringes zu betätigen.

8. Chirurgisches Instrument nach Anspruch 7, **dadurch gekennzeichnet, dass** die erste Vielzahl von Fingern (26) zur Aufnahme und zum Aussetzen eines proximalen Abschnitts des Anastomoseringes (14) konfiguriert ist, wobei der Ringaussetzmechanismus (20) ferner eine zweite Vielzahl von Fingern (28) aufweist, die zur Aufnahme und zum Aussetzen eines distalen Abschnitts des Anastomoseringes (14) konfiguriert sind.

9. Chirurgisches Instrument nach einem der Ansprüche 1 bis 6, **dadurch gekennzeichnet, dass** der längliche Schaft (15) einen proximalen Abschnitt und einen distalen Abschnitt aufweist, wobei der proximale Abschnitt an dem Handgriff (13) angebracht ist, wobei der Ringaussetzmechanismus (20) ein longitudinales Ende (28) und einen mittigen Abschnitt (32) aufweist, wobei der Ringaussetzmechanismus an dem distalen Abschnitt des länglichen Schaftes (15) positioniert ist, wobei der Ringaussetzmechanismus zur Aufnahme des Anastomoseringes (14) in einem komprimierten Zustand konfiguriert ist und wobei das flexible Verbindungselement (56) zum Bewegen des longitudinalen Endes (28) des Ringaussetzmechanismus (20) in Richtung auf den mittigen Abschnitt (32) des Ringaussetzmechanismus und zum Betätigen mindestens einen Teils des Ananstomoseringes (14) betreibbar ist.

## Revendications

1. Instrument chirurgical (10 ; 12) servant à implanter un dispositif de bague d'anastomose (14), comprenant :
(i) une poignée (13) ;
(ii) un mécanisme de déploiement de bague (16 ; 20) configuré pour recevoir une bague d'anastomose (14), dans lequel le mécanisme de déploiement de bague est adapté pour déplacer la bague d'anastomose (14) entre une position non actionnée généralement cylindrique et une position actionnée formant un rivet creux;
(iii) un mécanisme d'actionnement (22, 54 ; 24, 56) pouvant être actionné pour communiquer la force d'actionnement au mécanisme de déploiement de bague (16 ; 20) ;
(iv) une tige allongée (15) reliant la poignée (13) au mécanisme de déploiement de bague (16 ; 20) dans lequel la tige allongée comprend un ou plusieurs éléments rigides, incurvés (34) ; et
(v) un élément d'actionnement (22 ; 24) pouvant être actionné pour actionner le mécanisme de déploiement de bague (16 ; 20)
**caractérisé en ce que** l'élément d'actionnement (22 ; 24) est relié au mécanisme de déploiement de bague (16 ; 20) par un tube flexible (54 ; 56).

2. Instrument chirurgical selon la revendication 1, dans lequel le tube flexible (54 ; 56) comprend des fils tissés.

3. Instrument chirurgical selon la revendication 1 ou la revendication 2, dans lequel le mécanisme de déploiement de bague (20) comprend en outre un élément de non-actionnement stationnaire (32).

4. Instrument chirurgical selon la revendication 3, dans lequel le mécanisme de déploiement de bague (20) comprend en outre un élément de translation (26 ; 28).

5. Instrument chirurgical selon la revendication 4, dans lequel l'élément de translation (26 ; 28) se trouve dans un rapport articulé avec l'élément de non-actionnement stationnaire (32).

6. Instrument chirurgical selon la revendication 5, dans lequel l'élément de translation (26 ; 28) est configuré pour se déplacer vers l'élément stationnaire (32) en réponse à la force d'actionnement, ce qui entraîne l'articulation vers l'extérieur de l'élément de translation à partir de la tige allongée (15) pour déployer une partie de la bague d'anastomose (15).

7. Instrument chirurgical selon l'une quelconque des revendications précédentes, dans lequel le mécanisme de déploiement de bague (20) comprend une première pluralité de doigts (26 ; 28) configurée pour recevoir la bague d'anastomose (14), les doigts pouvant être déplacés d'une première position alignée de façon longitudinale avec la tige (15) jusqu'à une seconde position dans laquelle les doigts sont actionnés vers l'extérieur à partir d'un axe longitudinal de la tige allongée pour actionner au moins une partie de la bague d'anastomose.

8. Instrument chirurgical selon la revendication 7, dans lequel la première pluralité de doigts (26) est configurée pour recevoir et déployer une partie proximale de la bague d'anastomose (14), dans lequel le mécanisme de déploiement de bague (20) comprend en outre une deuxième pluralité de doigts (28) configurés pour recevoir et déployer une partie distale de la bague d'anastomose (14).

9. Instrument chirurgical selon l'une quelconque des revendications 1 à 6, dans lequel la tige allongée (15) comprend une partie proximale et une partie distale, dans lequel la partie proximale est reliée à la poignée (13), dans lequel le mécanisme de déploiement de bague (20) comprend une extrémité longitudinale (28) et une partie centrale (32), dans lequel le mécanisme de déploiement de bague est placé sur la partie distale de la tige allongée (15), dans lequel le mécanisme de déploiement de bague est configuré pour recevoir la bague d'anastomose (14) à l'état comprimé, et dans lequel l'élément de raccordement flexible (56) peut être actionné pour déplacer l'extrémité longitudinale (28) du mécanisme de déploiement de bague (20) vers la partie centrale (32) du mécanisme de déploiement de bague pour actionner au moins une partie de la bague d'anastomose (14).
